# EUROPEAN PATENT APPLICATION

(11) **EP 1 411 134 A1**
(43) Date of publication of application: **21.04.2004**
(21) Application number: 03291129.9
(22) Date of filing: 14.05.2003
(51) Int. Cl.: C12Q 1/68

(54) **Association of the H2 haplotype of the P2Y12 receptor with an increased risk for thrombosis and peripheral arterial disease**

(30) Priority: 15.10.2002 EP 02292543
(71) Applicant: INSTITUT NATIONAL DE LA SANTE ET DE LA RECHERCHE MEDICALE (INSERM), 75013 Paris (FR); ASSISTANCE PUBLIQUE - HOPITAUX DE PARIS, 75001 Paris (FR); Université René Descartes (Paris V), 75020 Paris Cedex 06 (FR)
(72) Inventor: Aiach, Martine, 92310 Sèvres (FR); Fontana, Pierre, 1006 Lausanne (CH); Gaussem, Pascale, 94240 l'Hay-Les-Roses (FR); Gandrille, Sophie, 75015 Paris (FR); Dupont, Annabelle, 59000 Lille (FR); Reny, Jean-Luc, 75013 Paris (FR)
(74) Representative: Bernasconi, Jean Raymond

(57) **Abstract**

The invention relates to an *in vitro* method for determining the risk of developing thrombosis in a subject, and to an *in vitro* method for determining sensitivity of a subject toward a thienopyridine therapy. Both methods involve identifying a particular haplotype of the P2Y₁₂ receptor gene.

## Description

The present invention relates to the identification of polymorphisms in the P2Y₁₂ receptor associated with platelet aggregation and thrombotic risk.

ADP (adenosine 5'-diphosphate) is one of the most important mediators of hemostasis and thrombosis (Storey et al., 2000). The effect of ADP on platelets is mediated by two P2Y receptors designated P2Y₁ and P2Y₁₂. Considerable evidence suggests that the P2Y₁₂ receptor plays a central role in the formation of hemostatic plugs and in the occurrence of arterial thrombosis (Mills et al., 1996 ; Cattaneo et al., 1999). The key role of P2Y₁₂ in arterial thrombosis is confirmed by the beneficial effect of the thienopyridine antithrombotic drugs, such as clopidogrel and ticlopidine that target the P2Y₁₂ receptor (Hollopeter et al., 2001 ; Quinn et al., 1999), in a variety of thrombotic diseases such as stroke, myocardial infarction and peripheral vascular disease (Quinn et al., 1999 ; and see CAPRIE Steering Committee. Lancet. 1996;348:1329-1339). Transduction of the ADP signal involves both a transient rise in Ca⁺⁺ mediated by the G_{q}-coupled P2Y₁ receptor (Jin et al., 1998a), and inhibition of adenylate cyclase mediated by the Gᵢ-coupled P2Y₁₂ receptor (Hollopeter et al., 2001 ; Foster et al., 2001). Simultaneous activation of the G_{q} and Gᵢ pathways by ADP is necessary for normal aggregation (Jin et al., 1998b ; Hechler et al., 1998a). Activation of the Gq pathway through P2Y₁ leads to platelet shape changes and a rapidly reversible wave of platelet aggregation (Jin et al., 1998a ; Hechler et al., 1998a), while activation of the Gᵢ pathway through P2Y₁₂ elicits slowly progressive and sustained platelet aggregation (Hechler et al., 1998b). The effect of P2Y₁₂ is not limited to inhibition of adenylate cyclase and a subsequent reduction in intracellular cAMP content. Indeed, it has been shown that P2Y₁₂ receptor activates glycoprotein (GP) Ilbllla integrin *via* a phosphoinositide 3- (PI 3-) kinase pathway (Kauffenstein et al., 2001) and/or another, unidentified G protein (Daniel et al., 1999). P2Y₁₂ thus appears to have a pivotal role in the irreversible wave of platelet aggregation that occurs on exposure to ADP.

The P2Y₁₂ gene, which encodes the 342-amino-acid receptor, was recently identified by two teams (Hollopeter et al., 2001 ; Foster et al., 2001 ; Zhang et al., 2001). Four patients have been described whose platelets, when exposed to ADP, undergo little or no reversible aggregation and show a decrease in the normal adenylate cyclase inhibition of prostaglandin E₁ (PGE₁)-stimulated platelets (Nurden et al., 1995 ; Cattaneo et al., 1992). In one patient, this phenotype was associated with a P2Y₁₂ gene abnormality (Hollopeter et al., 2001).

Considerable inter-individual variations in the concentration of ADP required to produce irreversible aggregation have been reported (Feng et al., 1999).

As inherited factors have a major role in platelet aggregation (O'Donnell et al., 2001), the inventors looked for sequence variations in the P2Y₁₂ gene that might explain the inter-individual variability of platelet aggregation responses to ADP.

The inventors identified three single-nucleotide polymorphisms (SNP) and a nucleotide insertion in the P2Y₁₂ receptor gene, which determined two haplotypes designated H1 and H2. The H2 haplotype was associated with higher maximal platelet aggregation in response to ADP.

The identification of a P2Y₁₂ receptor haplotype that is strongly associated with increased ADP-induced platelet aggregation has important clinical implications, particularly in screening for subjects at risk of thrombosis, especially atherothrombosis, and suboptimal responses to thienopyridine therapy.

Given the specificity of the P2Y₁₂ receptor in high risk peripheral arterial disease (PAD) patients and the gain of function of the H2 allele in platelet aggregation, the inventors further investigated the association of the P2Y₁₂ gene H2 haplotype with PAD in a case-control study.

They observed a strong association of P2Y₁₂ H2 haplotype with PAD.

Based on these results, the present invention provides a method for distinguishing H1 and H2 haplotypes in the P2Y₁₂ receptor, the latter being associated with a higher risk to develop thrombosis and/or with lower sensitivity toward a thienopyridine therapy.

This method is thus useful to determine the risk of developing thrombosis, especially atherothrombosis, in a subject.

This method is also useful to determine the sensitivity of a subject toward a thienopyridine therapy.

### P2Y₁₂ receptor

As above mentioned, the human P2Y₁₂ receptor has been cloned. Variants in other species has also been characterized.

| | Code | Access. number (SWISSPROT) |
|---|---|---|
| *Homo sapiens* | P2Y12_HUMAN | Q9H244 |
| *Macaca fascicularis* | P2Y12_MACFA | Q35KC3 |
| *Mus musculus* | P2Y12_MOUSE | Q9CPV9 |
| *Rattus norvegicus* | P2Y12_RAT | Q9EPX4 |

In the context of the present invention, the term "*P2Y*_{*12*} *receptor*" refers to the P2Y₁₂ receptor of any species, especially in human, but also in other mammals or vertebrates to which the methods of the invention can apply, if desired. The term *"subject"* thus refers to any human patient or any mammal or vertebrate.

The human P2Y₁₂ cDNA sequence is available on Genbank (access numbers: AF313449 and AY136754).

The inventors analyzed the P2Y₁₂ gene in a healthy population and found that it contained at least two exons separated by one intron approximately 1700 bp in length, located upstream of the ATG codon. Exon 2 encodes the entire 342-amino-acid protein. Because of a 6-nucleotide (nt) repeat sequence (table 1), the exact 3' end of exon 1 and 5' start site of exon 2 could not be determined.

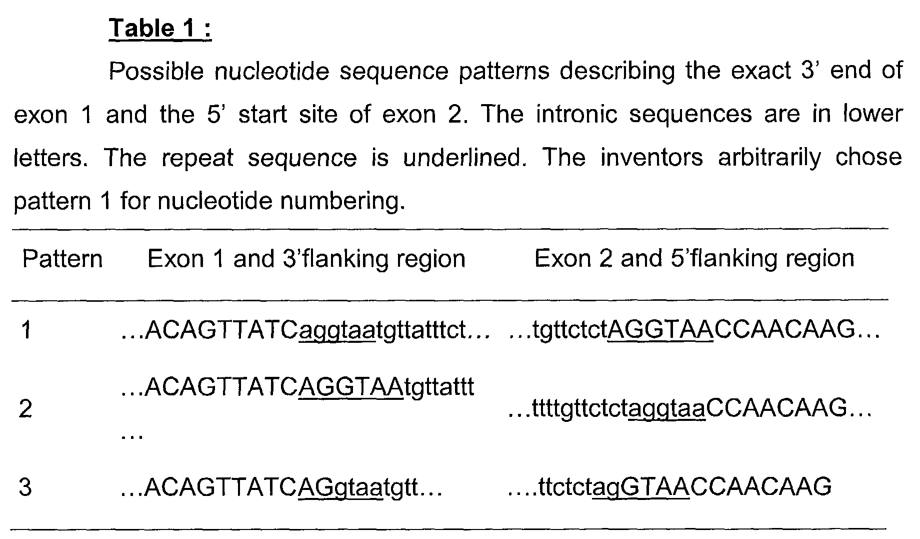

Table 1 describes three possible sequence patterns, pattern 3 being the most probable. However the inventors begun their work while arbitrarily choosing pattern 1 to number the polymorphisms that were studied. Consequently pattern 1 has been kept when numbering the polymorphisms described below.

According to pattern 1, sequence SEQ ID No 1 represents a 5' sequence of the intron, whereas sequence SEQ ID No 2 represents a 5' sequence of exon 2, which are the parts of interest in the P2Y₁₂ gene, according to the present invention. Both sequences show the H2 haplotype, as defined below. The A nucleotide of the starting codon ATG is nucleotide 17 in SEQ ID No 2.

### H1/H2 haplotypes

The inventors sequenced the P2Y₁₂ gene and found three single nucleotide polymorphisms (SNPs) and a single nucleotide insertion polymorphism of interest (Table 2). In the present invention, these polymorphisms are sometimes referred to as "the polymorphic positions of interest".

**Table 2 :**

| | | | | |
|---|---|---|---|---|
| Description of the polymorphisms detected in the study population. | | | | |
| The H1/H2 haplotype is defined by the i-C139T, i-T744C, i-ins801A and G52T polymorphisms. | | | | |

| Name of SNP | amino acid | Region | | Allele frequency |
|---|---|---|---|---|
| i-C139T | - | Intron | C: 86.2% | T: 13.8% |
| i-T744C | - | Intron | T: 86.2% | C:13.8% |
| i-ins801A | - | Intron | Insertion of an | A:13.8% |
| G52T | G/G | Exon 2 | G: 86.2% | T: 13.8% |
| C34T | N / N | Exon 2 | C: 72.5% | T: 27.5% |

Two variations were located 139 nt and 744 nt after the 5' intron (abbreviated as "i" herein) start site, consisting of a C to T (i-C139T) and a T to C (i-T744C) transition, respectively. Another polymorphism consisted of a single nucleotide insertion (A) at position 801 of the intron (i-ins801A). Two additional polymorphisms were found in exon 2, and consisted of a C to T transition (C34T) and a G to T transversion (G52T), respectively 17 nt and 35 nt after the first nucleotide of the ATG codon; neither modified the encoded amino acid (Asn⁶ and Gly¹², respectively). The C34T polymorphism was not associated with maximal aggregation in response to ADP.

As the i-C139T, i-T744C, i-ins801A and G52T polymorphisms were in complete linkage disequilibrium in a population of 100 healthy volunteers of caucasian origin, the inventors designated "H1" the haplotype of the major allele (a C in position 139, a T in position 744, and absence of the i-ins801A in the intron, and a G in position 52 of exon 2) and "H2" the haplotype of the minor allele (a T in position 139, a C in position 744, presence of the i-ins801A in the intron, and a T in position 52 of exon 2). The respective allele frequencies of haplotypes H1 and H2 were 86% and 14%. The allele frequencies of all the polymorphisms were in Hardy-Weinberg equilibrium.

Compared to the non carriers of the H2 allele (H1/H1), carriers of the H2 allele (H1/H2 or H2/H2) had a stronger ADP-stimulated platelet aggregation response *in vitro*, with different regulation of intracellular cAMP levels.

The isolated nucleic acid of the P2Y₁₂ receptor gene with the H2 haplotype is also part of the present invention.

A subject of the invention is thus an isolated nucleic acid encoding the P2Y₁₂ receptor, which nucleic acid comprises the P2Y₁₂ gene sequence with the simultaneous presence of T at position 139 of the intron, presence of C at position 744 of the intron, insertion of A at position 801 of the intron, and presence of T at position 52 of exon 2.

Such nucleic acid preferably comprises SEQ ID No 1 and/or SEQ ID No 2.

### Thrombosis risk

The present invention provides an *in vitro* method for determining the risk of developing thrombosis in a subject, which method comprises identifying polymorphisms of P2Y₁₂ receptor at positions 139, 744, and 801 of the intron (SEQ ID No 1), and at position 52 of exon 2 (SEQ ID No 2), wherein the simultaneous presence of T at position 139 of the intron, presence of C at position 744 of the intron, insertion of A at position 801 of the intron, and presence of T at position 52 of exon 2 are designated H2 haplotype and are indicative of a higher risk to develop thrombosis in comparison with a control subject, *i.e*. a subject showing no H2 haplotype on any of the alleles (*i.e*. a H1/H1 subject).

A higher risk to develop thrombosis means a significantly greater risk in carriers of the H2 allele than in non carriers.

Such a relative risk is frequently estimated through case-control studies and the use of the odds ratio (OR). An OR of 1.1 corresponds to an increase of 10% of the risk of developing disease and an OR of 2 corresponds to a 100 % increased risk.

The risk of developing thrombosis is connected with the induction of platelet aggregation.

Platelets are especially essential to arterial thrombosis and are also involved in the initiation and development of atherosclerotic lesions through adhesion to dysfunctional endothelium and release of growth factors and cytokines. The most important vascular targets of atherosclerotic disease include cerebral, coronary and lower limb arterial beds.

In the context of the present invention, the term "thrombosis" means formation of a clot in a blood vessel or in a heart cavity.

In a preferred embodiment, atherothrombosis is encompassed.

The terms "*atherothrombosis*", *"atherosclerosis"* and "*arterial thrombosis*" are synonymous.

Atherothrombosis is the central phenomenon of many thrombotic diseases such as stroke, myocardial infarction and peripheral arterial disease (PAD).

Concomitance of arterial thrombosis and atherosclerotic disease is of particular importance in peripheral arterial disease (PAD). Indeed, PAD is associated with a high risk of coronary and cerebrovascular atherosclerosis events and epidemiological studies have shown that 75% of PAD patients die of vascular causes (Ouriel et al., 2001).

Consequently, the identification of the H2 haplotype of the P2Y₁₂ receptor in a subject is further indicative of a higher risk to develop such thrombotic diseases, especially PAD.

Within the meaning of the invention, "thrombosis" as above defined encompasses other thrombotic states such as venous thrombosis and venous thromboembolic diseases, as well as thrombotic microangiopathy or intravascular disseminated coagulation.

### Sensitivity to thienopyridine therapy

The present invention further provides an *in vitro* method for determining sensitivity of a subject toward a thienopyridine therapy, which method comprises identifying polymorphisms of P2Y₁₂ receptor at positions 139, 744, and 801 of the intron (SEQ ID No 1), and at position 52 of exon 2 (SEQ ID No 2), wherein the simultaneous presence of T at position 139 of the intron, presence of C at position 744 of the intron, insertion of A at position 801 of the intron, and presence of T at position 52 of exon are designated H2 haplotype and, when present on at least one allele, are indicative of a lower sensitivity of the subject toward a thienopyridine therapy in comparison with a control subject, *i.e*. a subject with no H2 allele (*i.e*. a H1/H1 subject).

In the context of the invention, a "thienopyridine therapy" refers to the antithrombotic therapy currently used, which involves administration of thienopyridine drugs such as clopidogrel or ticlopidine, or any other agent that blocks the interaction between ADP and P2Y12.

The "sensitivity of a subject toward a thienopyridine therapy" refers to the variety of responses of a subject undergoing such therapy. A lower sensitivity toward the therapy corresponds to a poorer response, and therefore is not desired.

Determining the sensitivity of a subject toward a thienopyridine therapy is particularly interesting to help the physician design the therapeutic strategy that will be the most adapted to the subject, at an early stage.

### Identification of the presence of the H2 allele

The methods described above, *i.e*. the methods for determining the thrombosis risk and the methods to determine the sensitivity toward a thienopyridine therapy, involve the identification of the H1/H2 haplotype of the P2Y₁₂ receptor gene.

Such identification may be performed by any technique well known from the person skilled in the art.

In practicing the methods of the invention, an individual's polymorphic pattern with regard to the H1/H2 haplotype can be established by obtaining DNA from the individual and determining the sequence at the polymorphic positions of the P2Y₁₂ receptor gene interest.

The DNA may be obtained from any cell source. Non-limiting examples of cell sources available in clinical practice include blood cells, buccal cells, cervicovaginal cells, epithelial cells from urine, fetal cells, hair, or any cells present in tissue obtained by biopsy. Cells may also be obtained from body fluids, including without limitation blood, saliva, sweat, urine, cerebrospinal fluid, feces, and tissue exudates at the site of infection or inflammation. DNA may be extracted from the cell source or body fluid using any of the numerous methods that are standard in the art. It will be understood that the particular method used to extract DNA will depend on the nature of the source. In another embodiment, the analysis is achieved without extraction of the DNA, e.g. on whole blood (Mercier et al, 1990).

Determination of the sequence of the DNA at the polymorphic positions of the H1/H2 haplotype is achieved by any means known in the art. Numerous strategies for genotype analysis are available (Antonarakis et al., 1989 ; Cooper et al., 1991 ; Grompe, 1993). The strategies include, but are not limited to, direct sequencing, restriction fragment length polymorphism (RFLP) analysis, hybridization with allele-specific oligonucleotides, allele-specific PCR, PCR using mutagenic primers, ligase-PCR, HOT cleavage, denaturing gradient gel electrophoresis (DGGE), temperature denaturing gradient gel electrophoresis (TGGE), single-stranded conformational polymorphism (SSCP) and denaturing high performance liquid chromatography (Kuklin et al., 1997). Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology (see e.g. Little et al., 1996); and real-time quantitative PCR. Preferably, DNA from a subject is first subjected to amplification by polymerase chain reaction (PCR) using specific amplification primers. However several other methods are available, allowing DNA to be studied independently of PCR, such as the oligonucleotide ligation assay (OLI), rolling circle amplification (RCA) or the Invader™assay.

In a particular embodiment of the invention, it is provided an *in vitro* method for identifying at least one polymorphism of an haplotype of the P2Y₁₂ receptor associated with thrombosis in a subject or associated with lower sensitivity toward a thienopyridine therapy, which method comprises analyzing genomic DNA of a biological sample, in at least one of the regions of the P2Y₁₂ receptor gene, located around positions 139, 744 and 801 of the intron (SEQ ID No 1) and position 52 of exon 2 (SEQ ID No 2);
wherein the simultaneous presence of T at position 139 of the intron, presence of C at position 744 of the intron, insertion of A at position 801 of the intron, and presence of T at position 52 of exon 2 are designated H2 haplotype and, when present on at least one allele, are indicative of a higher risk to develop thrombosis or of a lower sensitivity toward a thienopyridine therapy, in comparison with a control subject.

The analysis may preferably comprise a step of amplification (*e.g.* by PCR) of said regions of the genomic DNA.

In a particular embodiment the analysis is undertaken on isolated (i.e. extracted) genomic DNA.

A preferred technique for identifying the polymorphisms of the P2Y₁₂ receptor includes sequencing.

### Kits

According to an aspect of the invention, the H1/H2 haplotype is detected by contacting the DNA of the subject with a nucleic acid probe, that is optionally labeled.

Primers may also be useful to amplify or sequence the portion of the P2Y₁₂ gene containing the polymorphic positions of interest.

Such probes or primers are nucleic acids that are capable of specifically hybridizing with a portion of the P2Y₁₂ gene sequence containing the polymorphic positions of interest. That means that they are sequences that hybridize with the nucleic acid sequence to which it refers under conditions of high stringency (Sambrook et al, 1989). These conditions are determined from the melting temperature Tm and the high ionic strength. Preferably, the most advantageous sequences are those which hybridize in the temperature range (Tm - 5°C) to (Tm - 30°C), and more preferably (Tm - 5°C) to (Tm - 10°C). A ionic strength of 6xSSC is more preferred. For instance, high stringency hybridization conditions correspond to the highest Tm, *e.g.*, 50 % formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., supra, 9.50-9.51). For hybridization with shorter nucleic acids, *i.e.*, oligonucleotides, the position of mismatches becomes more important, and the length of the oligonucleotide determines its specificity (see Sambrook et al., supra, 11.7-11.8). A minimum length for a hybridizable nucleic acid is at least about 10 nucleotides ; preferably at least about 15 nucleotides ; and more preferably the length is at least about 20 nucleotides.

Preferred probes or primers are described in the legend to the figures or in the examples below.

When the primers are mutagenic primers, the above rules for determining the hybridisation conditions are to be adapted. The sequences of mutagenic primers may indeed partially vary from the wild-type sequence, so as to introduce restriction site when a given allele is amplified. Furthermore such mutagenic primers are frequently 30 to 40 nucleotide-long.

The present invention further provides kits suitable for determining at least one of the polymorphism of the H1/H2 haplotype of the P2Y₁₂ receptor.

The kits may include the following components :
(i) a probe as above defined, usually made of DNA, and that may be pre-labelled. Alternatively, the probe may be unlabelled and the ingredients for labelling may be included in the kit in separate containers ; and
(ii) hybridization reagents : the kit may also contain other suitably packaged reagents and materials needed for the particular hybridization protocol, including solid-phase matrices, if applicable, and standards.

In another embodiment, the kits may include :
(i) sequence determination or amplification primers : sequencing primers may be pre-labelled or may contain an affinity purification or attachment moiety ; and
(ii) sequence determination or amplification reagents : the kit may also contain other suitably packaged reagents and materials needed for the particular sequencing amplification protocol. In one preferred embodiment, the kit comprises a panel of sequencing or amplification primers, whose sequences correspond to sequences adjacent to at least one of the polymorphic positions, as well as a means for detecting the presence of each polymorphic sequence.

In a particular embodiment, it is provided a kit which comprises a pair of nucleotide primers specific for amplifying all or part of the P2Y₁₂ gene comprising at least one of positions 139, 744 and 801 of the intron (SEQ ID No 1) and/or position 52 of exon 2 (SEQ ID No 2).

The below figures and examples illustrate the invention without limiting its scope.

### LEGENDS OF THE FIGURES :

Figure 1 is a schematic drawing showing the location of the primers and polymorphisms in the P2Y₁₂ gene.

A PCR product of approximately 3100 bp was obtained with primers E and J. Subsequent sequencing analysis was performed using primers 
B: 5'TCATGCCAGACTAGACCGAA3' (SEQ ID N° 3),
C: 5'ATCGATCGCTACCAGAAGACCACC3' (SEQ ID N° 4),
E: 5'GGCTGCAATAACTACTACTT3'(SEQ ID N° 5),
F: 5'AATTCCTTTCTGTCCTTAATT3' (SEQ ID N° 6),
G: 5'TAAATAGGTGAGGAGATGCTG3' (SEQ ID N° 7),
H: 5'TGCATTTCTTGTTGGTTACCT3' (SEQ ID N° 8),
J:5'GTCGTTTGTTTTGCTGCTAATA3' (SEQ ID N° 9),
K: 5'CATTGAGAATTTCAGCTCCC3' (SEQ ID N° 10),
 L: 5'ATACTAACTACTACAATGAAGAT3' (SEQ ID N° 11) and M: 5'CCTTACACCCTGAGCCAAAC3' (SEQ ID N° 12). ATG and TAA are start and stop codons, respectively. i-C139T, i-T744C, i-ins801A, C34T and G52T are the polymorphisms found in the study population.

Figure 2 is a graph showing maximal platelet aggregation responses to ADP in 98 healthy volunteers.

After a 3-min incubation period, platelets (250 x 10⁹/L in citrated PRP) were stimulated with 2 µM ADP. The concordance coefficient between aggregation values recorded at visit 1 and visit 2 (one week later) was 77% (*P*<0.001).

Figure 3 is a graph showing maximal aggregation in response to 2 µM ADP according to the P2Y₁₂ haplotype.

The average of the maximal aggregation values recorded at visits 1 and 2 in each volunteer was used for analysis. The median value was 34.7% in subjects carrying no H2 alleles (H1/H1, *n* = 74), 67.9% in subjects carrying one H2 allele (H1/H2, *n* = 21) and 82.4% in the three subjects carrying two H2 alleles (H2/H2) (*P*=0.0071).

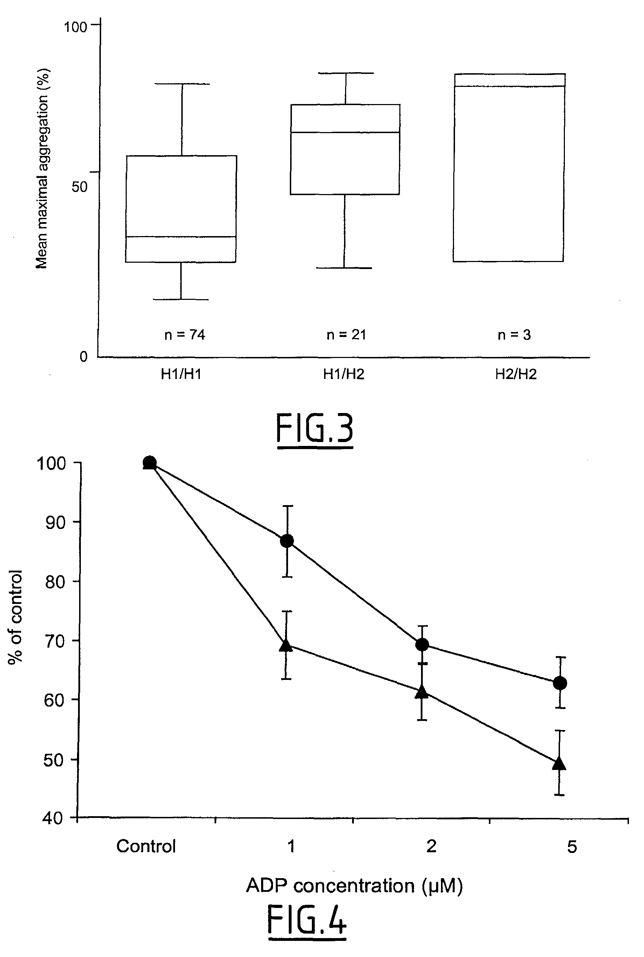

Figure 4 is a graph showing inhibition of iloprost-induced cAMP formation by ADP in carriers and non carriers of the H2 haplotype.

Iloprost (20 µg/µL final concentration) was added to citrated PRP 1 min after incubation at 37°C. Saline (control) or ADP at 1, 2 or 5 µM was added 1 min later. The reaction was stopped 3 min later and the cAMP concentration was determined using a commercial assay kit. Triangles and circles denote carriers and non carriers of the H2 haplotype, respectively. Means ± SEM.

### EXAMPLES :

### Example 1 : Identification of P2Y₁₂ gene sequence variations.

### Methods

### Subjects

Ninety-eight unrelated healthy Caucasian male volunteers aged from 18 to 35 years were recruited and investigated in the Clinical Investigation Center of Hôpital Européen Georges Pompidou. The volunteers were non smokers, had an unremarkable personal and familial medical history, and denied taking any medication for at least 10 days before blood collection. A physical examination and routine laboratory tests, including white blood cell, red blood cell and platelet counts, mean platelet volume, prothrombin time (PT), activated partial thromboplastin time (aPTT), plasma fibrinogen and C-reactive protein assays were performed before inclusion. Blood obtained from all the volunteers on day 0 (visit 1) and day 7 (visit 2) was subjected to aggregation tests. A third blood sample was obtained from a subset of 20 volunteers for platelet cAMP assay and comparison of maximal aggregation in citrated and in hirudin-anticoagulated platelet-rich plasma (PRP). All the subjects gave their written informed consent, and the study protocol was approved by the local ethics committee.

### Sample preparation

Venous blood was collected between 8:00 and 10:00 am, after an overnight fast, in tubes containing either 0.105 M sodium citrate (1 vol/9 vol) (BD Vacutainer®, Becton Dickinson, Le Pont de Claix, France) or 50 µg/mL lepirudine (Refludan®, Hoechst, Paris, France) using a 19-gauge needle and no tourniquet. The first 2 ml of blood was discarded. Platelet-rich plasma (PRP) was obtained by centrifugation at 1000 rpm for 10 min at room temperature. Autologous platelet-poor plasma, obtained by further centrifugation at 4000 rpm for 15 min, was used to adjust the platelet count of PRP to 250 x 10⁹/L.

### Platelet aggregation studies

Aggregation studies were performed within 2 hours after blood collection. Aggregation was measured at 37°C by using a photometric method on a four-channel aggregometer (Regulest® Amneville, France). A 280-µL aliquot of PRP was incubated for 3 min at 37°C and then was stirred at 1100 rpm for 2 min before adding 20 µL of saline or ADP (Sigma-Aldrich, St Quentin Fallavier, France) at 1, 2 or 5 µM (final concentration). The platelet aggregation response was recorded for 5 min.

### CAMP measurement

Platelet cAMP content was measured in 20 selected subjects by incubating 270 µL of PRP with 10 µL of a solution of the stable prostacyclin analog iloprost (20 µg/L, final concentration) (Ilomédine®, Schering-Plough) for 1 min, with stirring, then adding 20 µL of saline or ADP (1, 2 or 5 µM). The reaction was stopped 3 min later by adding 20 µL of 12% trichloroacetic acid. CAMP was then extracted and measured with an enzyme immunoassay (EIA) (Amersham, Pharmacia Biotech, Orsay, France) according to the manufacturer's instructions, without knowledge of the subject's P2Y₁₂ genotype.

### Genotyping studies

Genomic DNA was isolated from peripheral blood mononuclear cells by using the Qiamp Maxi Kit® (Qiagen, Courtaboeuf, France) according to the manufacturer's instructions.

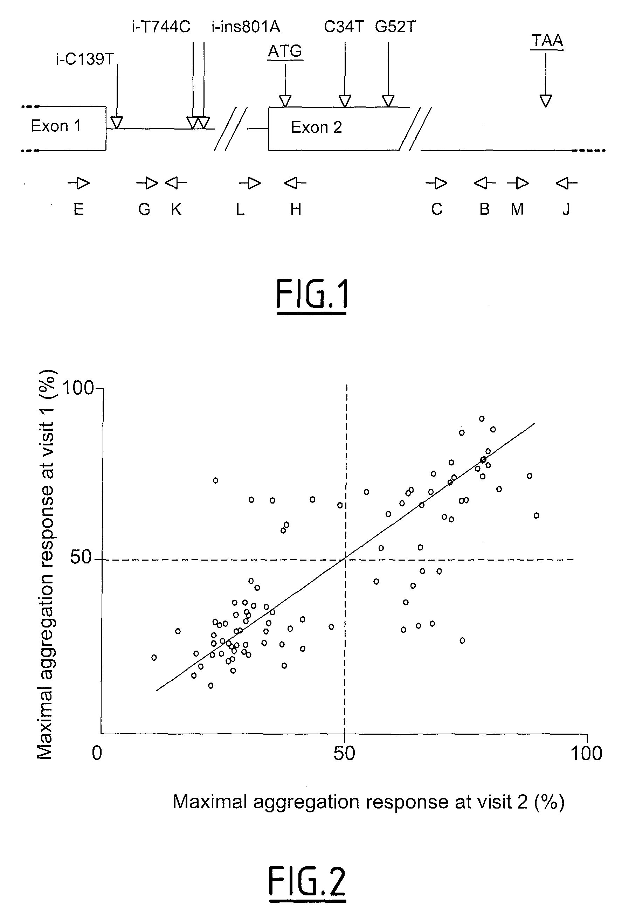

*P2Y*_{*12*} *receptor.* The inventors took advantage of the cDNA sequence reported by Hollopeter et al., (2001), which is available under GenBank accession number AF313449. Assuming that P2Y₁₂ gene organization was the same as that of seven other transmembrane-domain receptor genes, most of which comprise two exons separated by a single intron, the inventors performed a preliminary amplification experiment using two oligonucleotides, designated E and J, as sense and antisense primers; they were located at the 5' and 3' ends of the reported cDNA (figure 1). The amplification product was then purified with the Pre-Sequencing Kit (Amersham Pharmacia Biotech). Purification was followed by a cycle sequencing reaction using primers E, B and J. Subsequent sequencing using several other primers (G, H, K and L) (figure 1) allowed us to determine the nucleotide sequence of the amplification product. Sequence analysis was performed with an ABI Prism 3700 (Applera).

Screening of 40 subjects in a population is sufficient to identify polymorphisms with a frequency of 5% or more, with a confidence interval (CI) of 95%. Thus, a first series of 48 consecutive healthy volunteers were screened for P2Y₁₂ gene polymorphisms by sequencing the 58 nt of exon 1 with primer K, the 947 nt of its 3' flanking region with primers E and G, the 1274 nt of exon 2 with primers L, C and M, and the 570 nt of its 5' flanking region with primer H. The primers are described in figure 1. The P2Y₁₂ gene was then sequenced in the other 50 subjects by using primers targeting the identified polymorphisms.

PCR and sequencing results were analyzed without knowledge of the platelet aggregation results. Genotyping was repeated in ambiguous cases. All 98 subjects were successfully genotyped.

### Platelet RNA extraction and reverse transcription

Four milliliters of PRP (10⁹ platelets) was pelleted by centrifugation for 15 min at 4000 rpm. RNA was extracted with the RNeasy® kit (Qiagen) according to the manufacturer's instructions. Reverse transcription was performed using 10 units of RNasin™ ribonuclease inhibitor (Promega, Charbonnières, France), 100 units of Superscript II Rnase H- reverse transcriptase (Invitrogen) and 1.5 mM random hexanucleotides (Amersham Pharmacia biotech). The cDNA was stored at -20°C.

### Statistical analysis

Data are shown as means ± SEM, except for skewed variables, which are expressed as medians. Individual subjects' values obtained at visits 1 and 2 were compared using a concordance test (Lin, 1989) The *x*^{*2*} test was used to compare the observed allele and genotype frequencies with the Hardy-Weinberg equilibrium prediction. Trends across genotype groups were tested using the non parametric Kruskall-Wallis test. The association between the genotype and the maximal platelet aggregation response to ADP was tested, after adjustment for other variables, by using multivariate logistic regression models in which maximal aggregation < 50% was coded 0 and maximal aggregation ≥ 50% was coded 1. Considering the small number of subjects homozygous for the mutated allele (<7), whatever the polymorphism, these subjects were pooled together with heterozygotes for regression analysis.

Statistical tests were performed using the STATA 7.0 software package (Stata Corp, College Station, TX), and differences with P values <0.05 were considered statistically significant.

### Results

To assess the variability of ADP-induced platelet aggregation, the inventors tested two samples from each of 98 healthy volunteers, obtained one week apart. Figure 2 compares the maximal aggregation responses to 2 µM ADP in citrated PRP at the two visits. At least two phenotypic groups were identified. Maximal aggregation was below 50% at both visits in 47 subjects (48.0%). Moreover, 43 (91.5%) of the subjects in this subgroup had aggregation profiles consistent with a reversible primary phase. Conversely, maximal aggregation was above 50% at both visits in 29 subjects (29.6%), and 28 (96.5%) of these subjects had an irreversible second phase of ADP-induced aggregation. As maximal aggregation was stable between the two visits (*r*²=77%, *P*<0.001), we used the mean value for each subject (referred to below as maximal aggregation) for subsequent analyses.

The existence of these two distinct phenotypic groups of platelet aggregation, together with the stability of ADP responses over time, pointed to possible genetic control of the ADP effect on platelet aggregation. Thus, the inventors analyzed the P2Y₁₂ gene in the study population and found that it contained at least two exons separated by one intron approximately 1700 bp in length, located upstream of the ATG codon (figure 1). Exon 2 encodes the entire 342-amino-acid protein. Because of a 6-nucleotide (nt) repeat sequence (table 1), the exact 3' end of exon 1 and 5' start site of exon 2 could not be determined.

The inventors found three SNPs and a single nucleotide insertion polymorphism (Table 2) that defined the H1 and H2 haplotypes.

The H2 haplotype was associated with higher maximal aggregation in response to ADP, with median values of 34.7% in subjects carrying none of the H2 alleles (H1/H1, n=74), 67.9% in subjects carrying one H2 allele (H1/H2, n=21), and 82.4% in the three subjects carrying two H2 alleles (H2/H2) (Figure 3, P=0.0071). Multivariate logistic regression analysis indicated that the odds ratio (OR) of this association (OR 3.3, 95% CI: 1.1-10.4) did not change substantially after adjustment for age, body mass index, platelet count, mean platelet volume, white blood cell count, fibrinogen, PT, aPTT and the PI^{A1}/PI^{A2} genotype. The C34T polymorphism was not associated with maximal aggregation in response to ADP.

To investigate the association of the H2 haplotype and a possible splice variant of the intron located between exon 1 and 2, the inventors amplified and sequenced P2Y₁₂ cDNA obtained by reverse transcription of platelet mRNA from volunteers with the H1/H1 (n=3), H1/H2 (n=3) and H2/H2 (n=3) genotypes. Nucleotide sequencing revealed no variations, whatever the haplotype. Moreover, the sequence profile was consistent with amplification of both alleles in H1/H2 subjects, suggesting that both mRNA variants are transcribed (data not shown).

To further study the association between the H2 haplotype and ADP-stimulated platelet aggregation, the inventors randomly selected 10 carriers and 10 non carriers of the H2 haplotype. As ADP-induced platelet aggregation is considered to be dependent on the extracellular Ca⁺⁺ concentration (Packam et al., 1987), they measured maximal aggregation to 2 µM ADP in medium containing a low Ca⁺⁺ concentration (citrate-anticoagulated PRP) and in medium containing a physiological Ca⁺⁺ concentration (hirudin-anticoagulated PRP). With citrated blood, carriers and non carriers of the H2 allele had maximal aggregation of 71.5% ± 5.2% and 50.7% ± 7.2%, respectively (*P*=0.023). Similarly, in hirudin-anticoagulated blood, carriers and non carriers of the H2 allele had maximal aggregation of 53.8% ± 3.9% and 42.8% ± 3.4%, respectively (*P*=0.034).

To identify a possible link between the H2 haplotype and P2Y₁₂ regulation of adenylate cyclase, the inventors determined the capacity of ADP to inhibit iloprost-stimulated cAMP accumulation in platelets from the same 20 subjects. ADP inhibited cAMP formation in platelets from non carriers in a concentration-dependent manner, with 13%, 31% and 37% inhibition at 1, 2 and 5 µM ADP, respectively (figure 4), compared to 31%, 39% and 50%, respectively, in carriers of the H2 haplotype (*P*=0.028).

### Discussion

The inventors identified three single-nucleotide polymorphisms (SNP) and a nucleotide insertion in the P2Y₁₂ receptor gene, which determined two haplotypes designated H1 and H2. The H2 haplotype was associated with increased maximal platelet aggregation in response to ADP. This was related, at least in part, to differences in the mechanism regulating cAMP inhibition by ADP.

It has been shown in a large population study that ADP-induced platelet responses exhibit considerable variability as regards the ADP concentration required to produce a biphasic response with > 50% aggregation (Feng et al., 1999). In the present study, ADP-induced aggregation in the 98 healthy volunteers was stable between two measurements one-week apart, *i.e*. after renewal of most platelets. These results pointed to genetic control of ADP-induced platelet aggregation.

To further study the difference in the aggregation response to ADP between carriers and non carriers of the H2 haplotype, the inventors examined adenylate cyclase inhibition of iloprost-stimulated platelets. As shown in figure 4, a correlation was found between the H2 haplotype and the reduction in the intracellular cAMP concentration by ADP.

Data obtained with specific P2Y₁₂ receptor inhibitors such as the thienopyridines ticlopidine and clopidogrel confirm that P2Y₁₂ is the only known platelet ADP receptor responsible for adenylate cyclase inhibition and the subsequent reduction in intracellular camp (Geiger et al., 1999). Moreover, selective P2Y₁ receptor antagonists have no effect on ADP-induced adenylate cyclase inhibition (Jin et al., 1998a). Thus, the difference in the platelet cAMP concentration between carriers and non carriers of the H2 haplotype after ADP stimulation is a plausible explanation for the difference in maximal ADP-induced aggregation observed in these two groups of subjects, although we cannot rule out the involvement of adenylate cyclase-independent mechanisms (Kauffenstein et al., 2001).

The effect of the Ca⁺⁺ concentration on ADP-induced aggregation has been characterized with washed platelets (Packam et al., 1987). When hirudin is used as anticoagulant, the blood Ca⁺⁺ concentration is about 1.1 mM, and ADP induces much less granule content release than in citrated PRP (Mustard et al., 1975). However, although maximal aggregation was lower in hirudin-anticoagulated PRP in our study, the H2 allele remained associated with the aggregation response.

The molecular mechanism by which the H2 haplotype increases platelet aggregation in response to ADP remains to be determined. As the complete coding sequence of the P2Y₁₂ gene was screened in the present study population, an amino acid substitution affecting the protein structure can be ruled out. Moreover, cDNA analysis of both haplotypes showed normal sequences at the exon 1-exon 2 junction, ruling out a splice variant. Thus, an increase in the number of receptors on the platelet surface is most likely to explain the association between the H2 haplotype and platelet responsiveness to ADP. Polymorphisms in intronic and exonic enhancer regions, that augment transcription efficiency and the quantity of protein synthesized, have been described in other genes (Scohy et al., 2000; Watakabe et al., 1993).

### Example 2 : Association of the H2 haplotype with lower extremitiy peripheral arterial disease (PAD)

### Methods

Consecutive Caucasian male PAD patients younger than 70 years from the Paris area were recruited. PAD patients were defined as having symptomatic atherosclerotic disease of the lower limbs with an ankle/arm systolic blood pressure ratio < 0.90 or having a history of symptomatic lower limb atherosclerotic disease with previous surgical or endovascular revascularization. Control subjects had no history of arterial disease and were randomly selected, with age matching, among 703 caucasian males of a previously described control group designed for the study of genetic risk factors in vascular diseases (Arnaud et al., 2000). The calculation of the number of subjects needed was based on the allelic frequency of P2Y12 H2 haplotype as described in example 1, and was determined in order to detect an odds ratio of 2 or greater. With α=0.05 and β=0.20, 150 cases and 300 controls were needed for the study. Over a 2 year-period, 185 cases were included and matched to 331 controls. Each case was matched with 2 controls except for 38 cases who were each matched with one control. Case and control subjects gave written informed consent and the study protocol was approved by Paris-Cochin ethics committee.

Determination of the H2 allele of P2Y₁₂ gene was performed by polymerase chain reaction and sequence analysis as described in example 1.

### Results and discussion

Table 3 summarizes the characteristics of cases and controls (table 3).

| | | | |
|---|---|---|---|
| Table 3 : Subjects characteristics. Values are given as mean±SD or percentages for each group. LDL and HDL, low and high density lipoproteins ; BMI, body mass index. | | | |

| | Cases n=184 | Controls N=330 | *P* |
|---|---|---|---|
| Age (years) | 57.1±7.2 | 56.7±7.6 | 0.6 |
| Current and past smokers (%) | 97.8 | 47.0 | <0.0001 |
| Hypertension (%) | 43.5 | 16.1 | <0.0001 |
| Lipid lowering treatment (%) | 43.0 | 8.4 | <0.0001 |
| Total cholesterol (mmol/L) | 4.97±1.23 | 6.10±0.93 | <0.0001 |
| LDL cholesterol (mmol/L) | 3.04±1.03 | 3.94±0.87 | <0.0001 |
| HDL cholesterol (mmol/L) | 1.09±0.33 | 1.54±0.41 | <0.0001 |
| Triglycerides (mmol/L) | 1.96±1.35 | 1.41±0.87 | <0.0001 |
| Diabetes (%) | 24.5 | 8.5 | <0.0001 |
| Fasting blood glucose (mmol/L) | 6.5±2,4 | 5.9±0.7 | <0.0001 |
| Asymptomatic (%) | 10.3 | | |
| Intermittent claudication (%) | 75.6 | | |
| Critical ischemia (%) | 14.1 | | |
| Prior revascularization (%) | 55.4 | | |
| Ankle/arm systolic blood pressure ratio | 0.64±0.14 | | |
| Coronary heart disease (%) | 23.9 | | |
| Cereborvascular disease (%) | 10.4 | | |

The two groups differed significantly for prevalence of smoking status, hypertension and diabetes. Hypercholesterolemia was similar in both groups. Cases were more frequently treated with lipid lowering drugs and had lower total and LDL cholesterol values. Most PAD patients suffered from intermittent claudication and concurrent coronary artery or ischaemic cerebrovascular disease was present in 31%. Among PAD patients, 30% of the subjects were carriers of at least one H2 allele compared to 21% in the control group (*p*=0.03). Logistic regression results are described in table 4.

| | | | | | | |
|---|---|---|---|---|---|---|
| Table 4. Genotype frequencies of P2Y12 H1/H2 haplotype in cases and controls with univariate and multivariate OR for PAD. H1 / H2 and H2 / H2 groups are pooled together for OR calculation. OR of multivariate analysis is adjusted for hypertension, hypercholesterolemia, diabetes and tobacco use as defined in Methods. | | | | | | |

| | Cases n=184 | Controls n = 330 | Univariate OR (95% CI) | *P* value | Multivariate OR (95% CI) | *P* value |
|---|---|---|---|---|---|---|
| H1 / H1 (n) | 129 | 262 | | | | |
| H1 / H2 (n) | 51 | 59 | 1.6 (1.1 - 2.5) | 0.019 | 2.2 (1.3 - 3.7) | 0.004 |
| H2 / H2 (n) | 4 | 9 | | | | |

The H2 allele was associated with PAD with an OR=1.6 in univariate analysis. Upon adjustment on common cardiovascular risk factors including hypertension, hypercholesterolemia, diabetes and smoking status, the association was strengthened with an OR=2.2 (p=0.004).

This is the first study showing an association between a functional P2Y₁₂ gene polymorphism and atherosclerotic disease. P2Y₁₂ is an important step in the cascade of events leading to thrombotic complications in PAD patients.

The association of the H2 haplotype and PAD highlights the role of platelet in atherogenesis.

### REFERENCES

- Antonarakis et al. (1989), N. Engl. J. Med. 320:153-163
- Arnaud et al. (2000) Arterioscler Thromb Vasc Biol 20(3):892-8
- Cattaneo et al. (1992) Blood 80:2787-2796
- Cattaneo et al. (1999) Arterioscler Thromb Vasc Biol 19:2281-2285
- Cooper et al. (1991) Diagnosis of genetic disease using recombinant DNA, 3rd edition, Hum. Genet, 87:519-560
- Daniel et al., (1999) Thromb Haemost 82:1322-1326
- Feng et al. (1999) Arterioscler Thromb Vasc Biol 19:1142-1147
- Foster et al. (2001) J Clin Invest 107:1591-1598
- Geiger et al. (1999) Arterioscler Thromb Vasc Biol19:2007-2011
- Grompe, (1993) Nat. Genet. 5(2):111-7
- Hechler et al. (1998a) Blood 92:152-159
- Hechler et al. (1998b) Br J Haematol 103:858-866
- Hollopeter (2001) Nature 409:202-207
- Kuklin et al. (1997-98), Genet. Test, 1 (3):201-6
- Jin et al. (1998) J Biol Chem 273:2030-2034
- Jin et al. (1998) Proc Natl Acad Sci U S A 95:8070-8074
- Kauffenstein (2001) FEBS Lett 505:281-290
- Lin et al. (1989) Biometrics 45:255-268
- Little et al. (1996) Genet. Anal. 6:151
- Mercier et al. (1990) Acids Research, 18(19) :5908
- Mills et al. (1996) Thromb Haemost 76:835-856.
- Mustard et al. (1975) Am J Physiol 228:1757-1765
- Nurden et al. (1995) J Clin Invest 95:1612-1622
- O'Donnell et al. (2001) Circulation 103:3051-3056
- Ouriel et al. (2001) Lancet 358(9289):1257-64
- Packham et al. (1987) Blood 70:647-651
- Quinn et al. (1999) Circulation 100:1667-1672
- Sambrook et al. (1989) Molecular Cloning: A Laboratory Manual
- Scohy et al. (2000) Nucleic Acids Res 28:3743-3751
- Storey et al. (2000) Br J Haematol 110:925-934
- Watakabe et al. (1993) Genes Dev 7:407-418
- Zhang et al. (2001) J Biol Chem 276:8608-8615

## Claims

1. An *in vitro* method for determining a risk of developing thrombosis in a subject, which method comprises identifying polymorphisms of P2Y₁₂ receptor at positions 139, 744, and 801 of the intron (SEQ ID No 1), and at position 52 of exon 2 (SEQ ID No 2), wherein the simultaneous presence of T at position 139 of the intron, presence of C at position 744 of the intron, insertion of A at position 801 of the intron, and presence of T at position 52 of exon 2 are designated H2 haplotype and, when present on at least one allele, are indicative of a higher risk to develop thrombosis in comparison with a control subject without any H2 allele.

2. The method according to claim 1, wherein said thrombosis is an arterial thrombosis.

3. The method of claim 2, wherein the presence of the H2 haplotype on at least one allele is further indicative of a higher risk to develop peripheral arterial disease (PAD).

4. An *in vitro* method for determining sensitivity of a subject toward a thienopyridine therapy, which method comprises identifying polymorphisms of P2Y₁₂ receptor at positions 139, 744, and 801 of the intron (SEQ ID No 1), and at position 52 of exon 2 (SEQ ID No 2), wherein the simultaneous presence of T at position 139 of the intron, presence of C at position 744 of the intron, insertion of A at position 801 of the intron, and presence of T at position 52 of exon 2 are designated H2 haplotype and, when present on at least one allele, are indicative of a lower sensitivity of the subject toward a thienopyridine therapy, in comparison with a control subject without any H2 allele.

5. The method of claim 4, wherein the thienopyridine therapy is a therapy using ticlopidine or clopidogrel.

6. An *in vitro* method for identifying at least one polymorphism of an haplotype of the P2Y₁₂ receptor associated with thrombosis in a subject or associated with lower sensitivity toward a thienopyridine therapy, which method comprises analyzing genomic DNA of a biological sample, in at least one of the regions of the P2Y₁₂ receptor gene, located around positions 139, 744 and 801 of the intron (SEQ ID No 1) and position 52 of exon 2 (SEQ ID No 2); wherein the simultaneous presence of T at position 139 of the intron, presence of C at position 744 of the intron, insertion of A at position 801 of the intron, and presence of T at position 52 of exon 2 are designated H2 haplotype and, when present on at least one allele, are indicative of a higher risk to develop thrombosis or of a lower sensitivity toward a thienopyridine therapy, in comparison with a control subject.

7. The method according to claim 6, wherein the analysis is undertaken on genomic DNA that is extracted from the biological sample.

8. The method according to any of claims 6 or 7, wherein the analysis comprises a step of amplification of said region(s) of the genomic DNA.

9. The method according to any of claims 6 to 8, wherein the polymorphisms of the P2Y₁₂ receptor are identified by sequencing.

10. An isolated nucleic acid encoding the P2Y₁₂ receptor, which nucleic acid comprises the P2Y₁₂ gene sequence with the simultaneous presence of T at position 139 of the intron, presence of C at position 744 of the intron, insertion of A at position 801 of the intron, and presence of T at position 52 of exon 2.

11. A kit suitable for the methods according to any of claims 1 to 6, which kit comprises a pair of nucleotide primers specific for amplifying all or part of the P2Y₁₂ gene comprising at least one of positions 139, 744 and 801 of the intron (SEQ ID No 1) and/or position 52 of exon 2 (SEQ ID No 2).


SEQUENCE LISTING

<110>  INSERM

<120>  Identification of polymorphisms in the P2Y12 receptor associated with
platelet aggregation and thrombotic risk

<130>  BET 03P0500

<150>  EP 02292543.2
<151>  2002-10-15

<160>  12

<170>  PatentIn version 3.1

<210>  1
<211>  947
<212>  DNA
<213>  Homo sapiens

<400>  1
aggtaatgtt atttctacaa gtacttgaca aatatatgta tttacactct gatatgtgtg      60

tacatttttt ccaagtttct cttaaaattg taaaccttaa taactatcaa tgcaagcatg     120

tatttggaat aaaaattata attaaaatag aagtctaatt agtgatgtca gagacaattg     180

ttaattagcc tctttattaa gaaaaattat acaaagcata gaaataaaat ataaatggtc     240

ttagttactt tgcctcaagt ataaaatgag atttaaatac agataaaggg agctgaaatt     300

ctcaatgtca agaatacttg ttaaatatct tacagactac acaaaatgta tattcactaa     360

gtcgaatttc caaaacggtc agggatgaac taagaccaca cagcagtagc aggaaaggtt     420

aaaatcagtg atcttgtatt ggaaaacatt aggttttgtt tagcattatt ttaaagcgct     480

aaataggtga ggagatgctg aaaattgaag ccatactgtg acaacatgat tcttaatcgt     540

tttccttcca taattaagga cagaaaggaa ttccatggac attttgggga atttaagtgc     600

tacattcatt tatctaaata tcttttacac gaaagttatt ttttaaaatt tgttatttgt     660

actttcaata tatctctgat tattaagaat attttatata gaatcaattt cacttatctc     720

tggtgaaata aaaagattac aaacgtcatt tcaaattccc aagatgtaga tgccatatag     780

catattcaag tcacttgtta agttttcatt atagctgcct attgtggtaa taacctatat     840

tttattttag ctaccattac acactccata aatctggaaa gtatgccctg ttttgaggaa     900

tgccaactca tgaccatata tacacaggcc atttctgact cttattt                   947

<210>  2
<211>  130
<212>  DNA
<213>  Homo sapiens

<400>  2

aggtaaccaa caagaaatgc aagccgtcga caatctcacc tctgcgcctg gtaacaccag 60

tctgtgcacc agagactaca aaatcaccca ggtcctcttc ccactgctct acactgtcct 120

gtttttgtt 130

<210> 3
<211> 20
<212> DNA
<213> Artificial : primer

<400> 3
tcatgccaga ctagaccgaa 20

<210> 4
<211> 24
<212> DNA
<213> Artificial : primer

<400> 4
atcgatcgct accagaagac cacc 24

<210> 5
<211> 20
<212> DNA
<213> Artificial : primer

<400> 5
ggctgcaata actactactt 20

<210> 6
<211> 21
<212> DNA
<213> Artificial : primer

<400> 6
aattcctttc tgtccttaat t 21

<210> 7
<211> 21
<212> DNA
<213> Artificial : primer

<400> 7
taaataggtg aggagatgct g 21

<210> 8
<211> 21
<212> DNA
<213> Artificial : primer

<400> 8
tgcatttctt gttggttacc t 21

<210> 9
<211> 22
<212> DNA
<213> Artificial : primer

<400> 9
gtcgtttgtt ttgctgctaa ta 22

<210> 10
<211> 20
<212> DNA
<213> Artificial : primer

<400> 10
cattgagaat ttcagctccc 20

<210> 11
<211> 23
<212> DNA
<213> Artificial : primer

<400> 11
atactaacta ctacaatgaa gat 23

<210> 12
<211> 20
<212> DNA
<213> Artificial : primer

<400> 12
ccttacaccc tgagccaaac 20